(19) **Europäisches Patentamt**

**European Patent Office**

**Office européen des brevets**

(11) **EP 0 687 165 B1**

(12) **EUROPÄISCHE PATENTSCHRIFT**

(45) Veröffentlichungstag und Bekanntmachung des Hinweises auf die Patenterteilung:
**23.08.2000 Patentblatt 2000/34**

(51) Int. Cl.⁷: $A61F\ 2/34$, $B23G\ 1/00$

(21) Anmeldenummer: **95905022.0**

(22) Anmeldetag: **28.12.1994**

(86) Internationale Anmeldenummer:
**PCT/DE94/01551**

(87) Internationale Veröffentlichungsnummer:
**WO 95/18586 (13.07.1995 Gazette 1995/30)**

(54) **VERFAHREN ZUR HERSTELLUNG EINES GEWINDES MIT VERÄNDERLICH MODIFIZIERBAREM GEWINDEPROFIL UND ANWENDUNG DES VERFAHRENS**

PROCESS FOR PRODUCING A THREAD WITH A VARIABLE PROFILE AND APPLICATION OF THE PROCESS

PROCEDE DE PRODUCTION DE FILETS A PROFIL VARIABLE ET APPLICATION DU PROCEDE

(84) Benannte Vertragsstaaten:
**AT CH DE FR GB LI**

(30) Priorität: **02.01.1994 DE 4400001**

(43) Veröffentlichungstag der Anmeldung:
**20.12.1995 Patentblatt 1995/51**

(73) Patentinhaber:
**Hörmansdörfer, Gerd**
**D-31303 Burgdorf (DE)**

(72) Erfinder: **Hörmansdörfer, Gerd**
**D-31303 Burgdorf (DE)**

(56) Entgegenhaltungen:
**EP-A- 0 480 551      FR-A- 2 548 012**

## Beschreibung

Die Erfindung betrifft ein spezielles Verfahren für die spanende Herstellung von Gewinden gemäß Anspruch 1, bei welchen mindestens in einem Teilbereich ihrer Erstreckung die mehr oder weniger gleitende Beeinflussung bzw. Korrektur des Gewindeprofils erwünscht ist. Eine derartige Gestaltung kann für bestimmte Anwendungen vorteilhaft sein. Solche Anwendungen des Verfahrens werden in den Ansprüchen 14 und 19 beschrieben.

Die Erfindung betrifft auch eine einschraubbare Hüftgelenkpfanne gemäß den Ansprüchen 15 und 16.

Gewinde sind als konstruktive Elemente des allgemeinen Maschinenbaus weit verbreitet. Gewöhnlich sind Gewinde zylinderförmig gestaltet, daneben sind auch konische Gewinde z.B. für Ölfeldrohre verbreitet. Eine große Anzahl verschiedener Gewindeprofile sind bekannt und in Normen festgelegt. Üblicherweise ist das Gewindeprofil an einem Werkstück unveränderlich, das heißt, daß das Gewindeprofil am Gewindeanfang identisch mit dem am Gewindeende ist. Es sind jedoch Ausnahmen vorstellbar, bei denen eine zumindest in einem Teilbereich des Gewindes fließend sich ändernde Formgestalt des aus Gewinderille und Gewindezahn gebildeten Gewindeprofils von Vorteil sein könnte, z.B. um das Einführen eines Schraubengewindes in ein Mutterngewinde zu erleichtern.

Besondere geometrische Verhältnisse in Bezug auf das Gewinde bestehen jedoch vor allem bei Gewinden auf gekrümmten Flächen, wie sie insbesondere bei einschraubbaren künstlichen Hüftgelenkpfannen vorkommen. Hier sind hinsichtlich der Mantelform des Schalenkörpers z.B. hypo-, hemi- oder hypersphärische, konisch-sphärische, parabolische, toroidische, elliptische und ähnliche Geometrien bekannt. Bei spanenden Herstellungsverfahren für die Gewinde derartiger Schraubpfannen ergeben sich teilweise zwangsläufig fließend sich verändernde Verzerrungen des Gewindeprofils, welche in den meisten Fällen weder beabsichtigt noch erwünscht sind. Insbesondere bei der Benutzung von Gewindezähnen mit unsymmetrischen Flankenwinkeln ergibt sich das Phänomen, daß je nach Kipprichtung des resultierenden Gewindezahns die Zahnhöhe vom Pfannenäquator in Richtung zum Pfannenpol hin fließend zu- bzw. abnimmt, woraus sich dann am polnahen Gewindeanfang entweder viel zu große oder nahezu verkümmerte Gewindezähne ergeben. Im ersten Fall führen die extrem großen Gewindezähne dazu, daß zum Einschrauben der Pfanne sehr große Kräfte erforderlich werden, bzw. das Implantat nicht bis zum vollständigen Knochenkontakt eingeschraubt werden kann. Im zweiten Fall wird lediglich eine sehr dürftige Primärfixation zu erreichen sein. In beiden Fällen besteht die Gefahr des Auslockerns des Implantats, welche als Konsequenz eine nochmalige Operation des Patienten bedeuten würde.

Aus der Französischen Patentanmeldung 2 548 012 ist eine Schraubpfanne bekannt, deren äußere Form im Anmeldetext als zylindrisch-sphärisch beschrieben ist. Sie ist mit einem eingängigen Gewinde ausgestattet, welches sich über die gesamte äußere Länge erstreckt und dessen Gewindeprofil sich vom Pfannenäquator zum Pfannenpol hin fließend ändert. Diese Profiländerung kommt in einer Abmagerung der Zahnhöhe und einer zunehmenden Ungleichheit der Zahnflankenlängen in Richtung zum Pfannenpol hin zum Ausdruck. Das Gewinde ist als herkömmliches ISO-Gewinde mit einem eingeschlossenen Flankenwinkel von 60° gefertigt. Davon wird in den Zeichnungsfiguren sozusagen das Ergebnis des letzten Schnitts mit einem üblichen Werkzeug (z.B. Gewindeschneidplatte) gezeigt. Zwecks Erzielung einer in Bezug auf die Gewindezahnspitzen konstanten Gewindesteigung wird vorgeschlagen, während der Herstellung auf einer CNC-Maschine das Werkzeug auf einer Bahn mit einer sich bei jeder Umdrehung ändernden Steigung zu verfahren. Der geometrische Hintergrund zur Ermittlung der jeweiligen Steigung ist in Fig. 6 der genannten Anmeldung zeichnerisch dargestellt. Auf den ersten Blick erscheint die angegebene Vorgehensweise plausibel und ohne weiteres praktisch umsetzbar.

Bei näherer Analyse fällt zunächst auf, daß zwar die Gewindespitzen und der jeweilige Gewindegrund in der zweidimensionalen Darstellung auf Kreisbögen liegen, diese jedoch mit ihrem Drehpunkt nicht mit der Pfannenachse übereinfallen. Daher ist weder für den den Gewindegrund noch für den die Zahnspitzen einhüllenden geometrischen Mantel eine Kugelform realisiert. Eine präzise zeichnerische Darstellung mit einem CAD-System beweist dann, daß die ins Auge gefaßte Herstellungsmethode keinesfalls in der Lage ist, das dargestellte Gewindezahnprofil zu realisieren. Mit zunehmendem Abtauchen der Gewinderille zum Pfannenpol hin treten nämlich gravierende geometrische Effekte auf, welche sich in extremen Fluktuationen der Gewindezahnhöhe auswirken. In der von Hand gefertigten Zeichnungsfigur der Anmeldeschrift sind diese drastischen Fehler nicht sichtbar, weil sie durch eine Summierung einer größeren Zahl zeichnerischer Ungenauigkeiten ausgeglichen werden.

Tatsächlich ist es jedoch nicht möglich, mit dem unveränderlichen Gewindeprofil eines das Gewinde schneidenden Werkzeugs bei einem einzügigen Endschnitt mehreren voneinander unabhängigen Gesetzmäßigkeiten zu folgen.

Daneben ist das vorgeschlagene Gewinde mit ISO-Profil für den vorgesehenen Zweck sowieso ungeeignet, weil es einerseits dem Festigkeitsverhältnis zwischen dem Knochen und dem Implantatwerkstoff nicht Rechnung trägt und andererseits sehr hohe Einschraubkräfte beim Implantieren erfordert. Selbst bei Abänderung des Gewindeprofils, z.B. durch Verwendung eines Drehmeißels mit trapezförmigem Profil, und einer damit einhergehenden Verschmälerung der Gewindezähne wäre bei einer einzügigen Herstellungsweise der Gewindegrund durch Treppenstufen repräsentiert. Damit wären nicht nur Klemmeffekte beim Einschrauben verbunden, sondern auch eine unerwünschte Spaltenbildung im Kontaktbereich zur knöchernen Lagerfläche.

Es bestand daher die Aufgabe zur Schaffung einer spanend herstellbaren Hüftgelenkpfanne mit einem auf der gekrümmten Mantelfläche liegenden Gewinde mit unsymmetrischen Flanken des Gewindezahns und einer entlang der Gewindeerstreckung anpaßbaren Gewindezahnhöhe, sowie eines Verfahrens zur Herstellung solch eines speziellen Gewindes für diese und andere Anwendungen.

Die Aufgabe wird nach der Erfindung dadurch gelöst, daß die Gewinderille eines auf einer gekrümmten Mantelfläche liegenden Gewindes in mindestens zwei streifenförmige Teilflachen unterschiedlicher Steigung so aufgegliedert wird, daß sich die in der radialen Projektion gemessene Breite des Gewindegrundes entlang der Erstreckung des Gewindes fließend in der Weise ändert, daß die jeweils resultierende Gewindezahnhöhe der konstruktiven Vorgabe angepaßt ist. Mit der Erfindung wird ferner ein Verfahren zur Herstellung solch eines speziellen Gewindes zur Verfügung gestellt.

Dabei wird vorgeschlagen, die Gewinderille entlang der gleichen Kontur in dem zur Anpassung des Gewindeprofils vorgesehenen Bereich mindestens in zwei Herstellungsgängen mit mindestens zwei unterschiedlichen Steigungen zu bearbeiten. Dazu können wahlweise entweder das selbe Werkzeug oder zwei bzw. mehrere unterschiedliche Werkzeuge Verwendung finden. Soll nur ein Werkzeug eingesetzt werden, so müssen seine beiden Flanken der Formgestalt der beiden Gewindeflanken entsprechen. Die Erfindung eröffnet hier die Wahlmöglichkeit entweder ein Werkzeug zu verwenden, weiches in seinem Profil dem Profil der Gewinderille an ihrer engsten Stelle entspricht, oder ein Werkzeug, welches generell schmaler als die Gewinderille ist. Im zweiten Fall wird vorgeschlagen, für den Gewindeschnitt neben der Benutzung von mindestens zwei unterschiedlichen Steigungen für die Bearbeitungsgänge zusätzlich einen entsprechend angepaßten Offset-Wert einzubeziehen, um den Unterschied zwischen Werkzeug- und Gewinderillenbreite auszugleichen. Diese Vorgehensweise ist besonders vorteilhaft, weil damit z.B. eine kleinere Spitzenverrundung des Bearbeitungswerkzeugs ermöglicht wird. Dadurch kann der Gewindegrund feiner aufgelöst und der angestrebten gekrümmten Mantelfläche besser angepaßt werden.

Mit der Erfindung wird ferner die Wahlmöglichkeit angeboten, die Gewindebearbeitung mit mehr als zwei Herstellungsgängen, bzw. mit zwei oder mehreren Werkzeugen durchzuführen. Werden z.B. drei Herstellungsgänge angewandt, so wird empfohlen, bei einem der Herstellungsgänge im wesentlichen die eine Seite der Gewinderille, bei einem weiteren im wesentlichen die andere Seite der Gewinderille, und bei einem dritten Herstellungsgang im wesentlichen den Gewindegrund zu bearbeiten. Dabei wird für die die Seiten der Gewinderille und die jeweiligen Flanken der Gewindezähne schneidenden Werkzeuge der Maximal- bzw. Minimalwert der Steigung benutzt, während vorgeschlagen wird, für das im wesentlichen die Mitte des Gewindegrundes schneidende Werkzeug eine zwischen diesen beiden Werten liegende Steigung zu benutzen. Mit steigender Zahl der Bearbeitungsgänge bzw. Werkzeuge ist es so bei entsprechend kleiner Verrundung der jeweiligen Werkzeugspitze möglich, eine ungünstig grobe Verrundung des Gewindezahnfußes zu vermeiden und eine noch bessere Anpassung des Gewindegrundes an die angestrebte Kontur zu erzielen.

Das erfindungsgemäße Verfahren ist auf verschiedenen Maschinen mittels unterschiedlicher Zerspanungstechniken anwendbar, z.B. mittels Drehen, Drehfräsen oder Fräsen. Beim Gewindedrehen ist es üblich, die Gewinderille in mehreren Durchgängen mittels eines Drehmeißels zu bearbeiten. Der Herstellungsgang wird dann als Gewindeschneidzyklus bezeichnet. Bei einem einzigen Durchgang wird nur wenig Material abgetragen, dafür kann jedoch mit hohen Vorschüben gearbeitet werden. Demgegenüber kann ein Herstellungsgang beim Gewindefräsen aus einem einzigen Durchlauf bestehen, wofür sich jedoch aufgrund des erforderlichen kleinen Vorschubs in der Regel ein im Vergleich zum Drehen etwas höherer Zeitaufwand ergibt. Für das Schneiden des erfindungsgemäßen Gewindes sind moderne rechnergesteuerte Maschinen, sogenannte CNC-Maschinen, erforderlich. Im entsprechenden CNC-Programm muß die von dem jeweiligen Werkzeug relativ zum Werkstück zu beschreibende Bahn abgelegt sein. Im Programm ist ferner die beim Schneiden für den jeweiligen Herstellungsgang zu berücksichtigende unterschiedliche Steigung anzugeben. Zur Synchronisation der Herstellungsgänge bzw. Werkzeuge ist es erforderlich, die unterschiedlichen Startpunkte für die einzelnen Herstellungsgänge bzw. Schneidzyklen genau zu berechnen.

Die Berechnung zweier solcher Startpunkte soll anhand eines Beispiels für die drehtechnische Herstellung erklärt werden. Ein auf einer gekrümmten Mantelfläche zu schneidendes Gewinde soll sich von $z_3$ = -8 bis $z_4$ = -27 erstrecken. Es wird für ein Werkzeug A eine zu fahrende Steigung $s_1$ von 4 mm angenommen. Aufgrund der erforderlichen Synchronisationsstrecke (üblicherweise 2 x Gewindesteigung) von 8 mm wird der Startpunkt für den Gewindeschneidzyklus mit $z_1$ = 0 festgelegt, während der Endpunkt auf $z_4$ = -27 verbleibt. Es ergibt sich so ein Verfahrweg in z von 27 mm. Zur Erzielung einer bestimmten fließenden Anpassung der Gewindezahnhöhe wurde für ein zweites Werkzeug B die zu berücksichtigende Steigung $s_2$ auf 4,2 mm fest-

gelegt. Am Punkt $z_4 = -27$ sollen beide Werkzeuge synchron stehen. Der Startpunkt $z_2$ für das Werkzeug B errechnet sich dann wie folgt:

$$z_2 = \frac{[z_1 - z_4]}{s_1} + z_4$$

$$z_2 = \frac{[0 - (-27)] \cdot 4{,}2}{4} + (-27)$$

$$\underline{\underline{z_2 = +1{,}35}}$$

Im Falle der Benutzung eines Offset-Wertes für den axialen Parallelverschub eines einzigen Werkzeugs oder bei einer in keinem Punkt des Gewindes deckungsgleichen Position zweier Werkzeuge ist dieser Offset-Wert mit dem gemäß obiger Formel ermittelten z-Wert für den Startpunkt zu verrechnen.

Als besonders vorteilhaft wird vorgeschlagen, das oben näher erläuterte Verfahren zur Herstellung eines Gewindes mit veränderlich modifizierbarem Gewindeprofil mit einem vom Anmelder bereits zum Patent beantragten Verfahren zur Anpassung des Gewindegrundes an gekrümmte Flächen zu kombinieren. In der genannten Anmeldung (Europäische Patentanmeldung 91250274.7, Veröffentlichungsnummer 0 480 551 A1) wird ein Verfahren vorgestellt, mit welchem der Gewindegrund einem gekrümmten Verlauf nahezu perfekt anpaßbar ist, indem für die Herstellung Schneidwerkzeuge mit in Bezug auf den Gewindegrund unterschiedlichen Stirnwinkeln verwendet werden. Diese werden auf unterschiedlichen Bahnen, jedoch mit gleicher Steigung relativ zum Werkstück bewegt. Bei unterschiedlichen Zahnflankenwinkeln bzw. unsymmetrischen Zahnflanken sind die dann normalerweise fließend sich ändernden Zahnhöhen derartiger Gewinde dadurch auf die angestrebten Werte korrigierbar, daß die einzelnen Werkzeuge zusätzlich mit unterschiedlichen Steigungen relativ zum Werkstück verfahren werden.

Eine besondere Variante des hiermit vorgeschlagenen Verfahrens besteht darin, die Gewinderille einer derartigen einschraubbaren Hüftgelenkpfanne nacheinander unter Benutzung eines Offsets und verschiedener Steigungen mittels eines oder mehrerer Werkzeuge so abzufahren, daß in der Mine des Gewindegrundes zwischen den Gewindezähnen üblicher Höhe (z.B. zwischen 2,5 und 3,5 mm Höhe) ein weiterer sehr kleiner Gewindezahn (z.B. mit einer Höhe zwischen 0,2 und 1,5 mm, vorzugsweise zwischen 0,3 und 1,0 mm) gebildet wird. Diese Art der Bearbeitung dient der Schaffung eines Mikro-Makro-Gewindes auf dem Schraubpfannenmantel, wobei die normal großen Makrozähne im wesentlichen der Primärfixation, und die kleinen Mikrozähne im wesentlichen der Oberflächenvergrößerung und damit der Sekundärfixation dienen. Anhand der Festlegung der geometrischen Schnittbedingungen aus der Zahl der Bearbeitungsdurchläufe und deren jeweiligen Offset- bzw. Steigungswerten ist für solch ein Mikro-Makro-Gewinde festlegbar, inwieweit die Zahnhöhen sowohl der Makro- als auch der Mikrozähne entweder beibehalten oder fließend über die Kontur verändert werden sollen. Mit dem vorgeschlagenen Mikro-Makro-Gewinde wird eine sehr zuverlässige Verankerung des Implantats in der Knochenstruktur erzielt, weil dabei der Vorteil einer gröberen Gewindesteigung und der sich daraus ergebenden sehr großzügigen Breite und hohen Belastbarkeit der in den Gewinderillen stehenden Knochenstruktur mit dem Vorteil einer Oberflächenvergrößerung und der damit verbundenen Herabsetzung der spezifischen Grenzflächenbeanspruchung zwischen Implantat und Knochenstruktur in günstiger Weise verbunden ist.

Zum besseren Verständnis soll die Erfindung nachfolgend anhand der vier Zeichnungsfiguren näher erläutert werden.

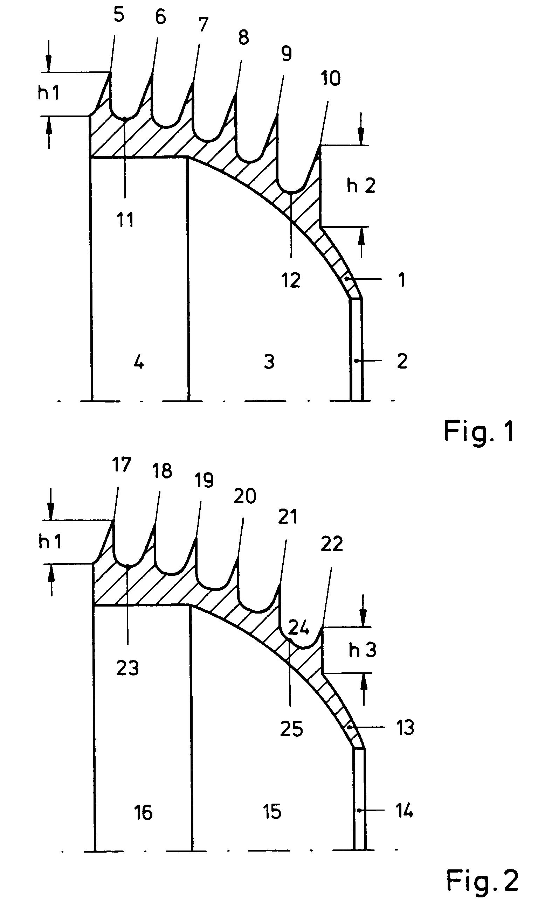

Fig.1 zeigt in einer geschnitten und halbseitig gezeichneten Darstellung ein etwas vereinfachtes Ausführungsbeispiel einer künstlichen hemisphärischen Hüftgelenkpfanne mittlerer Größe mit einem konventionell hergestellten Schraubgewinde in einem Maßstab von ungefähr 2,5 : 1. Dabei wurde für die Gewindesteigung ein Wert von 4 mm gewählt. Die Gewindezähne wurden leicht überhöht dargestellt, um die Details besser sichtbar machen zu können. Die Hüftgelenkpfanne (1) ist mit einem Bodenloch (2) versehen. Ihre innere Kontur gliedert sich in einen Kugelabschnitt (3) und einen zylindrischen Bereich (4) auf. Die sechs Gewindezähne (5, 6, 7, 8, 9 und 10) besitzen ein unsymmetrisches Profil. Ihre zum Pol der Pfanne zeigende Flanke weist einen Winkel von 0°, und ihre zum Äquator der Pfanne zeigende Flanke einen solchen von 20° auf. Dadurch ergibt sich ein Kippwinkel der Gewindezähne von 10° in Richtung zum Pfannenpol. Diese Kipprichtung der Gewindezähne erscheint für die angestrebte Verwendung bei einer Hüftgelenkpfanne besonders günstig, weil sich dadurch aufgrund der vorgegebenen Belastungsrichtung bessere Krafteinleitungsverhältnisse in das menschliche Becken erzielen lassen. Da das Gewinde mit nur einem Werkzeug und mit konstanter Steigung auf herkömmliche Weise geschnitten wurde, ist der Gewindegrund (11) in Äquatornähe mit dem Gewindegrund (12) in Polnähe identisch. Aus dem Kippwinkel der Gewindezähne und dem benutzten Herstellungsverfahren für das Gewinde resuliert eine fließende Veränderung der Gewindezahnhöhe. Es ist gut zu erkennen, daß die radial gemessene Gewindezahnhöhe $h_2$ in Polnähe etwa doppelt so groß ist, wie die Gewindezahnhöhe $h_1$ in Äquatornähe. Obwohl fließende Zu- oder Abnahmen der Gewindezahnhöhen bis zu einem gewissen Grad besonders bei Hüftgelenkpfannen durchaus gewollt sein können und dafür auch verschiedene Argumente ins Feld geführt werden, so ist doch eine solch starke Vergrößerung der Gewindezahnhöhe ausgesprochen nachteilig, weil damit unnötig tief in das Knochenmaterial eingegriffen wird. Derart große Gewindezähne am polnahen Gewindeanschnitt führen ferner insbesondere bei selbstschneidenden Schraubpfannen zu sehr hohen Einschraubkräften, da sich in dem Bereich des abrupten Anstiegs der Gewindezahnhöhe am Gewindebeginn die Zerspanungsarbeit beim Einschrauben in das Becken auf nur wenige Schneidkanten verteilt. Dadurch kann das Einbringen der Hüftgelenkpfanne bis zum vollständigen Knochenkontakt in Frage gestellt sein.

Fig.2 zeigt in einer mit Fig.1 identischen Darstellungsweise das vereinfachte und leicht verzerrte Ausführungsbeispiel einer erfindungsgemäßen Hüftgelenkpfanne mit Schraubgewinde. Die gezeigte Hüftgelenkpfanne (13) entspricht mit ihrem Bodenloch (14), ihrer inneren Kontur aus Kugelabschnitt (15) und zylinderförmigem Bereich (16) sowie der Zahl ihrer Gewindezähne jener aus Fig.1. Sowohl der äquatornahe Gewindezahn (17) mit seiner Form und dem Betrag seiner Höhe h 1, als auch der Gewindegrund (23) der benachbarten Gewinderille sind identisch mit dem entsprechenden Gewindezahn (5) bzw. dem benachbarten Gewindegrund (11) aus Fig. 1. Aufgrund der erfindungsgemäßen Bearbeitung des Gewindes mittels zweier Herstellungsgänge mit unterschiedlicher Steigung, wobei die mit 0° gezeichnete Flanke des Gewindezahns wie zuvor mit einer Steigung von 4 mm, jedoch abweichend davon die mit 20° gezeichnete Flanke des Gewindes mit einer Steigung von etwa 4,16 mm geschnitten ist, wird nunmehr für den polnahen Gewindezahn (22) eine radial gemessene Zahnhöhe h 3 realisiert, welche der Zahnhöhe h 1 des äquatornahen Zahns (17) entspricht. Es fällt auch auf, daß der Gewindegrund der polnahen Gewinderille (24) jetzt insgesamt breiter ist als der äquatornahe Gewindegrund (23) und einen von diesem abweichenden und besser dem Kugelmantel der Pfannenschale nahe kommenden Verlauf angenommen hat. Im Gewindegrund der polnahen Gewinderille (24) ist eine leicht außermittig liegende Kante (25) schwach angedeutet, welche sich aufgrund der erfindungsgemäßen Bearbeitungsweise längs der Gewinderille erstreckt und sich in ihrem abgewickelten Verlauf in Richtung zum Pfannenäquator langsam ausdünnt. Die maximale Höhe dieser Erhebung ist abhängig von der Geometrie bzw. Verrundung der verwendeten Werkzeugschneide, und damit durch entsprechende Auswahl an die konstruktiven Vorgaben für die Gestaltung des Gewindegrundes anpaßbar.

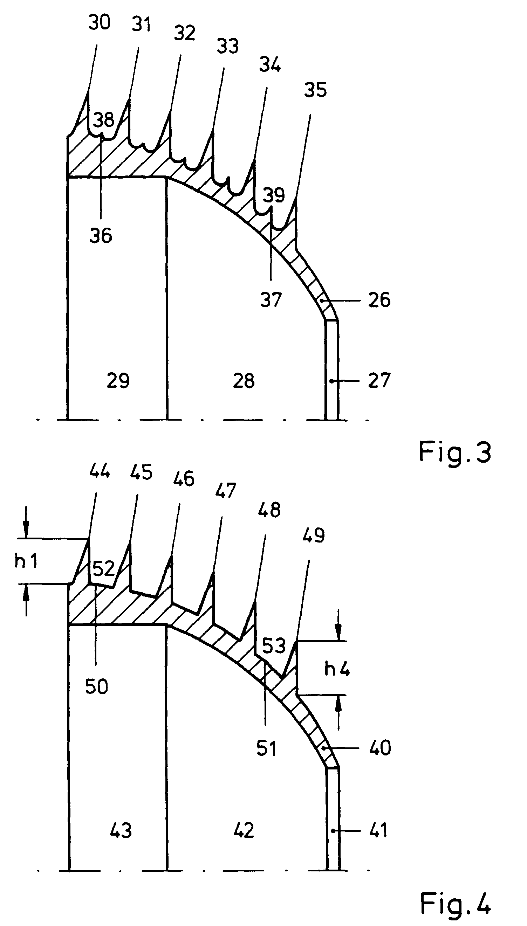

Das Beispiel einer besonderen Variante des erfindungsgemäßen Verfahrens ist in Fig.3 in Form einer parasphärischen Schraubpfanne mit Mikro-Makro-Gewinde dargestellt. Die Zeichnungsfigur zeigt in der schon aus den vorangehenden Figuren her bekannten Weise das Schnittbild einer Schraubpfanne (26) mit Bodenloch (27), deren Innenraum aus einem Kugelabschnitt (28) mit einem sich anschliessenden zylindrischen Bereich (29) gebildet ist. Die Schraubpfanne ist auf ihrem Mantel mit einem Makrogewinde ausgerüstet, von welchem sich im Schnittbild sechs Zähne (30, 31, 32, 33, 34, 35) ergeben. Abweichend von der Formgestalt einer Sphäre ist bei dem gezeigten Ausführungsbeispiel mittels des Gewindegrundes eine parasphärische Kontur realisiert, welche vor allem im Bereich nach dem polseitigen Gewindeanfang mit einer stärkeren Einschnürung gegenüber einem sphärischen Mantel versehen wurde, um hier Platz für die beim Eindrehen in das knöcherne Lager anfallenden Knochenspäne zu schaffen. In der dem Pfannenrand nahen Gewinderille (38) ist ein nicht sehr hoher Zahn (36) des etwa mittig in der Gewinderille umlaufenden Mikrogewindes zu erkennen. In der polnahen Gewinderille (39) hat der Zahn (37) des Mikrogewindes an Höhe zugenommen. Die stärkere Höhenzunahme des Mikrozahns bei nur leicht ansteigender radialer Höhe des Makrozahns resultiert aus der Bearbeitung mittels eines einzigen Werkzeugs, wobei in zwei Bearbeitungsdurchgängen zwei verschiedene Steigungen und eine Startpunktverschiebung zur Anwendung gekommen ist. Das Verfahren bietet generell die Möglichkeit, die Zahl der Bearbeitungsgänge unterschiedlicher Steigung und/oder Startpunktverschiebungen, bzw. die Steigungsdifferenzen selbst, zu erhöhen, bzw. anzupassen und zusätzliche Offset-Werte zu benutzen, um das geometrische Zerspanungsergebnis zu beeinflussen. Damit könnte z.B. ohne weiteres das Mikrogewinde auf eine gleichbleibende Zahnhöhe eingestellt werden.

Auch das in Figur 4 gezeigte Ausführungsbeispiel schließt sich in der gewählten Darstellungsform den zuvor gezeigten Figuren an. Es handelt sich dabei um eine hemisphärische Schraubpfanne (40), deren Kugelmantel durch Benutzung eines aus der Europäischen Patentanmeldung 91250274.7 bekannten Verfahrens für die Gewindeherstellung mittels Verwendung von fünf verschiedenen Schneidwerkzeugen mit den einzelnen Stirnkantenwinkeln von 2°, 14°, 25°, 35° und 44° nahezu perfekt nachgebildet wurde. Sowohl in der nahe dem Pfannenrand liegenden Gewinderille (52) als auch in der polnahen Gewinderille (53) ist die angestrebte gekrümmte Kontur derart sauber nachgebildet daß die mit dem Verfahren zwangsläufig sich ergebenden Dachkanten (50) bzw. (51) kaum sichtbar sind. Das genannte Verfahren wurde für das gezeigte Ausführungsbeispiel mit dem hiermit zum Patent angemeldeten Verfahren kombiniert, um die um 10° in Polrichtung geneigten Gewindezähne in ihrer Höhe zu beeinflussen. Dabei wurden die jeweiligen Steigungen für die fünf verschiedenen Schneidwerkzeuge so gewählt, daß das die polseitige Gewindeflanke schneidende Werkzeug mit der kleinsten, bzw. das die zum Pfannenrand zeigende Gewindeflanke schneidende Werkzeug mit der größten Steigung verfahren wurde. Der Steigungsunterschied zwischen der kleinsten und der größten Steigung wurde so festgelegt, daß die radial gemessene Gewindezahnhöhe h 1 des Gewindezahns (44) über die Gewindezähne (45, 46, 47, 48) bis zum Gewindezahn (49) auf einen leicht erhöhten Wert h 4 ansteigt, wobei dieser Anstieg wiederum daraus resultiert, daß versucht wurde, die mit einem Winkel von 20° geschnittene Flanke des Gewindezahns auf eine in etwa einheitliche Länge zu bringen. Im übrigen entspricht die Schraubpfanne mit ihrem Bodenloch (41) und ihrer aus Kugelabschnitt (42) und Zylinder (43) zusammengesetzten inneren Kontur den zuvor gezeigten Ausführungsbeispielen.

Aus dem letzten Ausführungsbeispiel ist zu sehen, daß das erfindungsgemäße Verfahren nicht darauf beschränkt ist, über den gesamten Erstreckungsbereich des Gewindes eine einheitliche Gewindezahnhöhe zu realisieren. Im Gegenteil eröffnet es dadurch zahlreiche Variationsmöglichkeiten, daß entweder durch entsprechende Festlegung der zur Anwendung kommenden unterschiedlichen Gewindesteigungen exakt bestimmbare Zu- oder Abnahmen der Gewindezahnhöhen verwirklicht werden können, oder diese Modifikationen partiell in Teilbereichen der Gewindeerstreckung bzw. auch gleitend mittels der Programmierung einer kontinuierlich sich ändernden Steigung einbringbar sind. Damit wird mit der Erfindung ein sehr flexibel einsetzbares Mittel für die optimale Anpaßbarkeit von Spezialgewinden, insbesondere von gekrümmten Spezialgewinden, zur Verfügung gestellt.

**Patentansprüche**

1. Verfahren zur spanenden Herstellung eines Gewindes mit sich mindestens in einem Teilbereich im wesentlichen kontinuierlich veränderndem Gewindeprofil, wobei das Gewinde mindestens in diesem Teilbereich mit mindestens zwei Bearbeitungsgängen bzw. -zyklen bearbeitet wird, dadurch gekennzeichnet, daß dabei unterschiedliche Steigungen benutzt werden.

2. Verfahren gemäß Anspruch 1, dadurch gekennzeichnet, daß ein Bearbeitungsgang bzw. -zyklus im wesentlichen die eine Flanke des Gewindezahns sowie einen Teil des benachbarten Gewindegrundes, und ein weiterer Bearbeitungsgang bzw. -zyklus im wesentlichen die andere Flanke des Gewindezahns sowie einen Teil des dieser anderen Flanke benachbarten Gewindegrundes betrifft.

3. Verfahren gemäß einem der Ansprüche 1 oder 2, dadurch gekennzeichnet, daß das Gewinde in dem genannten Teilbereich mit drei verschiedenen Bearbeitungsgängen bzw. -zyklen bearbeitet wird, wovon mit dem dritten Bearbeitungsgang bzw. -zyklus im wesentlichen die Mine des Gewindegrundes bearbeitet wird, und dabei eine Steigung benutzt wird, welche zwischen den für die beiden anderen Bearbeitungsgänge bzw. -zyklen benutzten Steigungen liegt.

4. Verfahren gemäß einem der Ansprüche 1, 2 oder 3, dadurch gekennzeichnet, daß für die Bearbeitungsgänge bzw. -zyklen das selbe Werkzeug verwendet wird.

5. Verfahren gemäß einem der Ansprüche 1, 2 oder 3, dadurch gekennzeichnet, daß für mindestens zwei Bearbeitungsgänge bzw. -zyklen unterschiedliche Werkzeuge verwendet werden.

6. Verfahren gemäß einem der Ansprüche 1, 2 oder 3, dadurch gekennzeichnet, daß für sämtliche Bearbeitungsgänge bzw. -zyklen unterschiedliche Werkzeuge benutzt werden.

7. Verfahren gemäß einem der Ansprüche 1 bis 6, dadurch gekennzeichnet, daß das Gewinde mindestens in einem Teilbereich mit einer kontinuierlich sich ändernden Steigung geschnitten ist.

8. Verfahren gemäß einem der Ansprüche 1 bis 7, dadurch gekennzeichnet, daß mittels der für die Bearbeitungsgänge bzw. -zyklen programmierten Werkzeugbahnen ein Gewindegrund (23, 24, 25 / 52, 53) hergestellt ist, welcher einer vorgegebenen gekrümmten Mantelfläche weitgehend angeglichen ist.

9. Verfahren gemäß einem der Ansprüche 1 bis 3 oder 6 bis 8, dadurch gekennzeichnet, daß der Gewindegrund aus an Kanten (50, 51) zusammenstoßenden Teilflächen gebildet und mit verschiedenen Werkzeugen mit in Bezug auf den Gewindegrund unterschiedlichen Stirnschneidenwinkeln auf unterschiedlichen Bahnen bearbeitet wird.

10. Verfahren gemäß einem der Ansprüche 1 bis 9, dadurch gekennzeichnet, daß für mindestens zwei Bearbeitungsgänge bzw. -zyklen für das selbe Werkzeug oder für mindestens zwei verschiedene Werkzeuge eine gegenseitige Startpunktverschiebung in der Steigungsachse (z-Achse) benutzt wird.

11. Verfahren gemäß Anspruch 10, dadurch gekennzeichnet, daß der verschobene Startpunkt wie folgt bestimmt ist:

$$z_2 = \frac{[z_1 - z_4]}{s_1} + z_4$$

mit

$z_1$ = Startpunkt für Werkzeug oder Bearbeitungsgang bzw. -zyklus A
$z_2$ = Startpunkt für Werkzeug oder Bearbeitungsgang bzw. -zyklus B
$z_4$ = Synchronpunkt für Werkzeuge oder Bearbeitungsgänge bzw. -zyklen A und B

$s_1$ = Steigung für Werkzeug oder Bearbeitungsgang bzw. -zyklus A

$s_2$ = Steigung für Werkzeug oder Bearbeitungsgang bzw. -zyklus B

**12.** Verfahren gemäß einem oder mehreren der vorgenannten Ansprüche, <u>dadurch gekennzeichnet</u>, daß das Gewinde mit einer Anzahl von Bearbeitungsgängen bzw. -zyklen und/oder Werkzeugen unter Heranziehung jeweiliger Offset-Werte für einen axialen Parallelverschub in der Steigungsachse geschnitten wird.

**13.** Verfahren gemäß Anspruch 12, <u>dadurch gekennzeichnet</u>, daß die jeweiligen Offset-Werte mit dem Startpunkt des Bearbeitungsgangs bzw. -zyklus in der Steigungsachse (z-Achse) verrechnet werden.

**14.** Verfahren gemäß einem oder mehreren der vorgenannten Ansprüche, zur spanenden Herstellung eines Gewindes (17, 18, 19, 20, 21, 22) auf einer Hüftgelenkpfanne (13) mit gekrümmter äußerer Kontur und in Richtung zum Pfannenpol hin gekipptem Gewindezahn.

**15.** Einschraubbare Hüftgelenkpfanne mit gekrümmter äußerer Kontur, welche mit einem Gewinde zur zementfreien Verankerung im Acetabulum versehen ist, <u>dadurch gekennzeichnet</u>, daß mindestens in einem Teilbereich des Gewindes die rechte Flanke des Gewindezahns und der sich anschließende Bereich des Gewindegrundes, und/oder die linke Flanke des Gewindezahns und der sich dieser linken Flanke anschließende Bereich des Gewindegrundes in mindestens zwei separaten Bearbeitungsgängen bzw. -zyklen so bearbeitet sind, daß der Gewindegrund in seiner Abwicklung in mindestens zwei streifenförmige Teilflächen unterschiedlicher Steigung aufgegliedert ist.

**16.** Einschraubbare Hüftgelenkpfanne (13) mit gekrümmter äußerer Kontur, welche mit einem Gewinde (17, 18, 19, 20, 21, 22) zur zementfreien Verankerung, im Acetabulum versehen ist, <u>dadurch gekennzeichnet</u>, daß das Gewinde mit unterschiedlichen Steigungen geschnitten ist, und die in der radialen Projektion gemessene axiale Breite des Gewindegrundes (24) entlang der Erstreckung des Gewindes vom Pfannenpol zum Pfannenrand hin mindestens in einem Teilbereich sich fließend ändert.

**17.** Einschraubbare Hüftgelenkpfanne gemäß Anspruch 16, <u>dadurch gekennzeichnet</u>, daß in der Mine des zwischen zwei größeren Gewindezähnen (Makrozähnen) (34, 35) gebildeten gebildeten Gewindegrundes (39) ein weiterer Gewindezahn (Mikrozahn) (37) gebildet ist, welcher wesentlich kleiner, vorzugsweise um mindestens 50% kleiner ist, als der größere Gewindezahn (Makrozahn).

**18.** Einschraubbare Hüftgelenkpfanne gemäß Ansprüchen 16 oder 17, <u>dadurch gekennzeichnet</u>, daß der Gewindegrund (52, 53) in seiner Abwicklung durch winkelmäßig in Stufen (50, 51) aufgelöste streifenförmige Teilflächen der gekrümmten Mantelfläche der Hüftgelenkpfanne (40) weitgehend angeglichen ist.

**19.** Einschraubbare Hüftgelenkpfanne mit gekrümmter äußerer Kontur, welche mit einem Gewinde zur zementfreien Verankerung im Acetabulum versehen ist, <u>dadurch gekennzeichnet</u>, daß sie mit einem Verfahren nach einem oder mehreren der Ansprüche 1 bis 13 hergestellt ist.

**Claims**

**1.** Process for the cutting production of a thread, said thread essentially having a steadily changing thread profile over at least a portion of its extension, wherein said thread at least within this portion is produced using at least two machining passes or cycles, <u>characterized in that</u> different pitches are applied for this.

**2.** Process as in claim 1, <u>characterized in that</u> one of the machining passes or cycles is used for the production essentially of one of the thread tooth flanks and a part of the adjoining thread bottom, and another machining pass or cycle is used essentially for the production of the other thread tooth flank and a part of the thread bottom adjoining this other flank.

**3.** Process according to claims 1 or 2, <u>characterized in that</u> the thread within said portion is produced using three different maching passes or cycles, wherein the third machining pass or cycle is used essentially for the production of the middle of the thread bottom, using a pitch for this, which is between the pitches of the other two machining passes or cycles.

**4.** Process according to one of the claims 1, 2 or 3, <u>characterized in that</u> the same machining tool is used for the machining passes or cycles.

**5.** Process according to one of the claims 1, 2 or 3, <u>characterized in that</u> different machining tools are used for at least two machining passes or cycles.

**6.** Process according to one of the claims 1, 2 or 3, <u>characterized in that</u> different machining tools are used for all machining passes or cycles.

**7.** Process according to one of the claims 1 to 6, <u>characterized in that</u> the thread at least in a portion of its extension is produced using a continuously changing pitch.

**8.** Process according to one of the claims 1 to 7, <u>characterized in that</u> the tool paths for the machining passes or cycles are programmed to produce a thread bottom (23, 24, 25 / 52, 53), which is adapted in a high degree to a given curved outer surface.

**9.** Process according to one of the claims 1 to 3 or 6 to 8, <u>characterized in that</u> the thread bottom is consisting from part surfaces adjoining at rims (50, 51), said thread bottom being produced by means of different cutting tools having frontal cutting angles differing in relation to the thread bottom, using different tool paths.

**10.** Process according to one of the claims 1 to 9, <u>characterized in that</u> at least for two machining passes or cycles for the same tool or at least two different tools, a reciprocal starling point offset is applied within the thread pitch axis (z-axis).

**11.** Process according to claim 10, <u>characterized in that</u> the starting point offset is calculated as follows:

$$z_2 = \frac{[z_1 - z_4]}{s_1} + z_4$$

with

$z_1$ = starting point for tool or machining pass or cycle A
$z_2$ = starting point for tool or machining pass or cycle B
$z_4$ = convergent point for tools or machining passes or cycles A and B
$s_1$ = thread pitch for tool or machining pass or cycle A
$s_2$ = thread pitch for tool or machining pass or cycle B.

**12.** Process according to one or several of the preceding claims, <u>characterized in that</u> the thread is produced using a number of machining passes or cycles and/or machining tools, wherein respective offset values are applied for an axial parallel adjustment within the thread pitch axis (z-axis).

**13.** Process according to claim 12, <u>characterized in that</u> the respective offset values are settled against the machining pass or cycle starting point within the thread pitch axis (z-axis).

**14.** Process according to one or several of the preceding claims, for the cutting production of a thread (17, 18, 19, 20, 21, 22) on a hip joint socket (13) having a curved outer surface and a thread tooth inclined towards the socket pole.

**15.** Screw-in hip joint socket having a curved outer contour provided with a thread for cement-less anchoring in the acetabulum, <u>characterized in that</u> at least in a portion of the thread extension the right flank of the thread tooth and an adjoining pad of the thread bottom, and/or the left flank of the thread tooth and a part of the thread bottom adjoining this left flank are produced using at least two individual machining passes or cycles in such a way that the thread bottom in its development is divided into at least two strip-like surfaces of different pitch.

**16.** Screw-in hip joint socket (13) having a curved outer contour provided with a thread (17, 18, 19, 20, 21, 22) for cement-less anchoring in the acetabulum, <u>characterized in that</u> the thread is produced using different pitches, the axial width of the thread bottom (24) along the extension of the thread between the socket pole and the socket rim, measured in the radial projection, having at least within a portion of the thread extension a continous variation.

**17.** Threaded hip joint socket according to claim 16, <u>characterized in that</u> in the middle of the thread bottom (39) between two larger thread teeth (macro-teeth) (34, 35) a further thread tooth (micro-tooth) (37) is formed, which is considerably smaller, preferably at least 50% smaller than the larger thread tooth (macro-tooth).

**18.** Threaded hip joint socket according to claims 16 or 17, <u>characterized in that</u> the thread bottom (52, 53) in its development is adapted in a high degree to the curved outer socket surface (50, 51), using strip-like part surfaces angularly resolved in steps (50, 51).

**19.** Screw-in hip joint socket having a curved outer contour provided with a thread for cement-less anchoring in the acetabulum, <u>characterized in that</u> it is produced using a process according to one or several of the claims 1 to 13.

**Revendications**

**1.** Procédé pour la fabrication par enlèvement de copeaux d'un filet avec un profil de filetage se modifiant pour l'essentiel continuellement au moins dans une zone partielle, le filet étant usiné au moins dans cette zone partielle avec au moins deux opérations ou cycles d'usinage, <u>caractérisé par en ce que</u> différants pas sont utilisés.

**2.** Procédé selon la revendication 1, <u>caractérisé en ce que</u>, une opération ou cycle d'usinage concerne pour l'essentiel un flanc de la dent de filetage ainsi qu'une partie du fond de filet voisin, et une autre opération ou cycle d'usinage concerne pour l'essentiel l'autre flanc de la dent de filetage ainsi qu'une partie du fond de filet voisin de cet autre flanc.

**3.** Procédé selon la revendication 1 ou 2, <u>caractérisé en de que</u> le filet est usiné dans la dite zone partielle avec trois différentes opérations ou cycles d'usinage, le centre du font de filet étant usiné pour l'essentiel avec la troisième opération ou cycle d'usinage en utilisant à cela un pas qui se situe entre les pas utilisés pour les deux autres opérations ou cycles d'usinage.

**4.** Procédé selon une des revendications 1, 2 ou 3, <u>caractérisé en ce que</u> le même outil est utilisé pour les opérations ou cycles d'usinage.

**5.** Procédé selon une des revendications 1, 2 ou 3, <u>caractérisé en ce que</u> différents outils sont utilisés pour au moins deux opérations ou cycles d'usinage.

**6.** Procédé selon une des revendications 1, 2 ou 3, <u>caractérisé en ce que</u> différents outils sont utilisés pour toutes les opérations ou cycles d'usinage.

**7.** Procédé selon une des revendications 1 à 6, <u>caractérisé en ce que</u> le filet est taillé au moins dans une zone partielle avec un pas se modifiant continuellement.

**8.** Procédé selon une des revendications 1 à 7, <u>caractérisé en ce que</u>, un fond de filet (23, 24, 25 / 52, 53), lequel est largement adapté à une surface latérale courbée prédéfinie, est fabriqué au moyen des chemins de l'outil programmés pour les opérations ou cycles d'usinage.

**9.** Procédé selon une des revendications 1 à 3 ou 6 à 8, <u>caractérisé en ce que</u> le fond de filet est formé des surfaces partielles se heurtant aux arêtes (50, 51) et est usiné sur différents chemins avec différents outils avec différents angles de dépouille de face par rapport au fond de filet.

**10.** Procédé selon une des revendications 1 à 9, <u>caractérisé en ce que</u>, pour au moins deux opérations ou cycles d'usinage, pour le même outil ou pour au moins deux outils différents, on utilise un décalage de point de démarrage réciproque dans l'axe de pas (axe z).

**11.** Procédé selon la revendication 10, <u>caractérisé en</u>

<u>ce que</u> le point de démarrage décalé est déterminé comme suit:

$$z_2 = \frac{[z_1 - z_4]}{s_1} + z_4$$

avec

$z_1$ = point de démarrage pour l'outil ou l'opération ou cycle d'usinage A
$z_2$ = point de démarrage pour l'outil ou l'opération ou cycle d'usinage B
$z_4$ = point des synchronisation pour les outils ou opérations ou cycles d'usinage A et B
$s_1$ = pas pour l'outil ou l'opération ou cycle d'usinage A
$s_2$ = pas pour l'outil ou l'opération ou cycle d'usinage B

**12.** Procédé selon une ou plusieurs revendications susnommées, <u>caractérisé en ce que</u> le filet est taillé avec un nombre d'opérations ou cycles d'usinage et/ou des outils en utilisant des valeurs offset respectives pour un déplacement parallèle axial dans l'axe de pas.

**13.** Procédé selon la revendication 12, <u>caractérisé en ce que</u> les valeurs offset respec-tives sont compensées avec le point de démarrage de l'opération ou cycle d'usinage dans l'axe de pas (axe z).

**14.** Procédé selon une ou plusieurs revendications susnommées, pour la fabrication par enlèvement de copeaux d'un filet (17,18,19,20,21,22) sur une cupule cotyloïde (13) avec un contour extérieur courbé et une dent de filetage basculée vers le pôle de la cupule.

**15.** Cupule cotyloïde vissable avec un contour extérieur courbé, laquelle est pourvue d'un filet pour l'ancrage sans ciment dans l'acétabule, <u>caractérisé en ce qu'</u>on usine, au moins dans une zone partielle du filet, le flanc droit de la dent de filetage et la zone avoisinante du fond de filet et/ou le flanc gauche de la dent de filetage et la zone avoisinante de ce flanc gauche du fond de filet, dans au moins deux opérations ou cycles d'usinage séparées, de telle sorte que le fond de filet soit divisé dans son déroulement en au moins deux surfaces partielles striées de pas différent.

**16.** Cupule cotyloïde vissable (13) avec contour extérieur courbé, laquelle est pourvue d'un filet (17,18,19,20,21,22) pour l'ancrage sans ciment dans l'acétabule, <u>caractérisé en ce que</u> le filet est taillé avec différents pas, et la largeur axiale du font de filet (24) mesurée dans la projection radiale le long de l'extension du filet de pôle de la cavité vers le bord de la cavité se modifie en continu au moins dans une zone partielle.

17. Cupule cotyloïde vissable selon la revendication 16, caractérisé en ce que, au centre du fond de filet (39) formé entre les deux plus grandes dents de filetage (macro-dents) (34,35), une autre dent de filetage (micro-dent) est formée, laquelle étant sensiblement plus petite, de préférence d'au moins 50%, que la plus grande dent de filetage (macrodent).

18. Cupule cotyloïde vissable selon les revendications 16 ou 17, caractérisé en ce que le fond de filet (52,53) est largement adapté, dans son déroulement, à la surface latérale courbée de la cupule cotyloïde (40), par des surfaces partielles striées réduites en paliers (50,51).

19. Cupule cotyloïde vissable avec contour extérieur courbé, laquelle est pourvue d'un filet pour l'ancrage sans ciment dans l'acétabule, carctérisé en ce qu'elle est fabriquée avec un procédé selon une ou plusieurs des revendications 1 à 13.

Fig. 1

Fig. 2

Fig.3

Fig.4